# EUROPÄISCHE PATENTSCHRIFT

(11) **EP 1 918 620 B1**
(45) Veröffentlichungstag und Bekanntmachung des Hinweises auf die Patenterteilung: **19.08.2009**
(21) Anmeldenummer: 07019522.7
(22) Anmeldetag: 05.10.2007
(51) Int. Cl.: F16K 15/14

(54) **Rückschlagventil, insbesondere für medizinische Anwendungen**
Non-return valve, in particular for medical applications
Clapet anti-retour, en particulier pour des applications médicales

(30) Priorität: 30.10.2006 DE 202006016730 U
(43) Veröffentlichungstag der Anmeldung: 07.05.2008
(73) Patentinhaber: Filtertek B.V., County of Limerick (IE)
(72) Erfinder: Costello, Kieran, Cullenagh Ballina Co. Tipperary (IE); Carmondy, Colm, Trieneragh Duagh Co. Kerry (IE); Casey, Brendan, Drombane Thurles Co. Tipperary (IE)
(74) Vertreter: Brose, D. Karl

(56) Entgegenhaltungen:
- EP-A- 1 703 183
- DE-A1- 2 819 900
- DE-A1- 4 304 949
- DE-U1- 9 319 810
- DE-U1-8202004 009 35
- DE-U1-1202004 009 52
- US-A- 4 188 978
- US-A- 5 025 829

## Beschreibung

Die Erfindung betrifft ein Rückschlagventil, insbesondere für medizinische Anwendungen, mit einem ersten Schlauchanschlussgehäuse und einem zweiten Schlauchanschlussgehäuse und einer zwischen den beiden Schlauchanschlussgehäusen angeordneten Membranscheibe auf flexiblem Werkstoff, welche bei Überdruck in einem Eingangskanal des ersten Schlauchanschlussgehäuses von einem ringförmigen, einen mit dem Eingangskanal verbundenen Einlassraum umgebenden Ventilsitz abhebbar ist und die bei Überdruck in einem Auslasskanal des zweiten Schlauchanschlussgehäuses sicher und in Minimalzeiten auf den Ventilsitz andrückbar ist, wobei die Membranscheibe in ihrem äußeren Umfangsbereich mit einem Ringwulst versehen ist, der in einander gegenüberliegenden Ringnuten der Schlauchanschlussgehäuse aufgenommen ist, die Membranscheibe radial außerhalb des Ventilsitzes mit zu einem Auslassraum führenden Öffnungen versehen ist, und wobei die den Öffnungen gegenüberliegende Wandung des zweiten Schlauchanschlussgehäuse mit mit dem Auslasskanal in Verbindung stehenden Ausnehmungen versehen ist.

Ein derartiges Rückschlagventil ist nach einem früheren Vorschlag der Anmelderin aus dem DE-GBM 20 2004 009 358.8 bekannt.

Derartige Rückschlagventile werden in der Medizintechnik in den Leitungen von Infusionssystemen, Diagnoseausrüstungen, Spritzen, intravenösen Schlauchleitungen und dergleichen benötigt. Folglich müssen derartige Rückschlagventile sehr sicher schließen und die Schließzeit darf nur Bruchteile von Sekunden betragen, um damit jeglichen Rückfluss mit unerwünschten Stoffen belasteten Flüssigkeiten zu vermeiden. Gleichzeitig muss die Herstellung nicht nur extrem wirtschaftlich, sondern auch statistisch sehr genau sein, da derartige Ventile in der Medizintechnik ausschließlich als Einwegartikel verwendet werden und daher aus Kostengründen in automatisierter Weise in großen Stückzahlen hergestellt werden müssen. Darüber hinaus bestehen sehr strenge gesetzliche Vorschriften, um eine gleichbleibende und einheitliche Funktionssicherheit zu gewährleisten, was beispielsweise in Deutschland durch den Technischen Überwachungsverein genau geprüft wird. Erst nach entsprechend positiven Ergebnissen erfolgt die Freigabe derartiger Rückschlagventile für die medizinische Anwendung.

Das Ventil nach der oben erwähnten Gattung verbessert ein Rückschlagventil, welches nach einem anderen früheren Vorschlag der Anmelderin aus dem Europäischen Patent 0 612 537 bekannt ist. Dieses bekannte Ventil erfüllt zwar die oben erwähnten Anforderungen durchaus, da bei dieser Konstruktion die den Ringwulst am Umfangsbereich der Membranscheibe aufnehmenden Nuten derart gestaltet sind, dass bei der Montage der beiden Schlauchanschlussgehäuse auf die Membranscheibe radial gerichtete Zugkräfte aufgebracht werden, so dass hierdurch eine Einstellung der Zugspannung in der Membran erfolgen kann, welche ein noch schnelleres und sichereres Ansprechen des Rückschlagventils gewährleistet. Beim praktischen Einsatz dieses bekannten Ventils hat es sich jedoch nun gezeigt, dass es bei höheren Drücken auf der Eingangsseite des Ventils oder hohen Differentialdrücken vorkommen kann, dass die in der Membran vorgesehenen Öffnungen sich aufgrund dieses Überdrucks an die gegenüberliegende Wandung des Auslassraums anlegen können und somit das Ventil unbeabsichtigt schließt.

Um dies zu vermeiden, ist bei der Weiterentwicklung des Ventils der eingangs genannten Art vorgesehen, dass die den Öffnungen gegenüberliegende Wandung des zweiten Schlauchanschlussgehäuses der Auslassseite des Rückschlagventils mit mit dem Auslassdurchlass in Verbindung stehenden Ausnehmungen versehen ist, welche als Ringkanal mit einem Radius ausgebildet sind, der dem Radius entspricht, auf welchem die Öffnungen der Membranscheibe angeordnet sind. Hierdurch wird erreicht, dass selbst, falls sich unter Überdruck die Membranscheibe vollständig an die Wandung des Auslassraums anlegen sollte, dennoch der gewünschte Durchflussquerschnitt auf jeden Fall frei bleibt, indem die Strömung zur Auslassseite über den Ringkanal erfolgt.

Der praktische Einsatz dieses Ventils hat jedoch gezeigt, dass diese Ausgestaltung bei hohen Drücken möglicherweise nicht ausreicht, da sich die Membran derart stark verformen kann, dass sie die summierte verfügbare offene Fläche verringert, durch welche die Flüssigkeit strömen kann. Eine weitere Schwierigkeit, die sich bei hohen Drücken ergibt, besteht darin, dass, nachdem der hohe Druck entlastet wurde, die Membran überbeansprucht gewesen sein kann und nicht mehr eine ausreichende Dichtfunktion erfüllt, wobei gleichzeitig der Öffnungsdruck erheblich abgesenkt wird.

Ausgehend von einem Rückschlagventil der eingangs genannten Art liegt daher der Erfindung die Aufgabe zugrunde, das oben beschriebene Ventil derart weiterzuentwickeln, dass diese Schwierigkeiten vermieden werden.

Bei einem Rückschlagventil der eingangs genannten Art wird diese Aufgabe im Wesentlichen dadurch gelöst, dass die Ausnehmungen durch Stützflächen für die Membranscheibe voneinander getrennt sind, und dass die Ausnehmungen durch schmale tiefe Nuten mit dem Auslasskanal verbunden sind. Hierdurch wird der Vorteil erzielt, dass selbst, falls sich unter hohen Drücken die Membranscheibe vollständig der Wandung des Auslassraums annähert, diese Bewegung unmittelbar und in definierter Weise von den Stützflächen zwischen den Ausnehmungen aufgenommen wird, so dass weder eine Verringerung des verfügbaren Strömungsquerschnitts noch eine Überdehnung der Membran eintreten kann. Durch die schmalen tiefen Nuten, welche die Ausnehmungen mit dem Auslasskanal verbinden, wird erreicht, dass die Stützflächen ohne Verringerung des Strömungsquerschnitts entsprechend großflächig ausgebildet werden können.

Bei einer bevorzugten Ausführungsform nach der Erfindung sind die Stützflächen bis zum Außenrand des Auslassraums reichend ausgebildet.

Im Einzelnen ist es bevorzugt, dass die Stützflächen sich in Richtung des Außenrandes des Auslassraums verbreiternd ausgebildet sind. Durch diese letztgenannten bevorzugten Merkmale ist es möglich, besonders großflächige Stützflächen vorzusehen.

Im Einzelnen ist es ferner bevorzugt, dass die Stützflächen Innenenden aufweisen, welche innerhalb des Radius' des gegenüberliegenden Ventilsitzes angeordnet sind.

Eine bevorzugte Weiterbildung nach der Erfindung besteht darin, dass die Innenenden mit etwa auf deren Radius angeordneten Vorsprüngen versehen sind, die an der Membranscheibe anliegen und diese in Richtung des Einlassraums halten bzw. vorspannen. Hierdurch ist es möglich, mit geringem Aufwand das Rückschlagventil nach der Erfindung für höhere Öffnungsdrücke auszugestalten, was beispielsweise bei der Verwendung im Zusammenhang mit Spritzpumpen von Vorteil ist, da hier sichergestellt werden muss, durch den Höhenunterschied zwischen Patient und Spritzpumpe diese nicht einfach leer laufen kann, wenn das Ventil bereits aufgrund der geodätischen Höhe öffnen könnte. Durch die innerhalb des Radius' des auf der anderen Seite der Membranscheibe liegenden Ventilsitzes und durch die Größe der durch die Vorsprünge in der Membranscheibe erzeugte gegensinnige Vorspannung wird daher eine einfache Möglichkeit geschaffen, Ventile unterschiedlicher Öffnungsdrücke herzustellen, indem entweder der Radius, auf welchem diese Vorsprünge innerhalb des Ventilsitzes liegen, oder die Höhe der Vorsprünge und somit die Größe der gegensinnigen Vorspannung geändert wird. Unter geringsten Formkosten lässt sich dies leicht dadurch erreichen, dass lediglich eine Hälfte der zur Herstellung des zweiten Schlauchanschlussgehäuses verwendeten Spritzform entsprechend geändert wird. Da die Vorsprünge ferner lediglich an den Innenenden der Stützflächen vorgesehen sind, wird hierdurch die Strömung durch das geöffnete Ventil in keiner Weise behindert.

Eine besonders bevorzugte Ausführungsform kann dadurch geschaffen werden, dass die Stützflächen mit seitlich in einem spitzen Winkel abstehenden Zusatzflächen versehen sind, deren radiale Außenenden innerhalb des Radius' liegen, auf welchem die Öffnungen in der Membranscheibe angeordnet sind. Hierdurch wird die mögliche Anlagefläche der Membranscheibe bei Überdruck nochmals vergrößert, ohne dass hierdurch die Strömung durch die Öffnungen in der Membranscheibe behindert wird.

Besonders bevorzugt ist es ferner, dass die der Membranscheibe gegenüberliegende Oberfläche der Stützflächen und/oder Zusatzflächen konkav gewölbt ausgebildet ist. Hierdurch wird ein schonendes Anliegen der Membran bei Überdruck an den Stützflächen und Zusatzflächen gewährleistet, da deren konvexe Ausgestaltung der natürlichen Wölbung der Membran in diesem Zustand entspricht.

Im Einzelnen ist es ferner bevorzugt, dass die Nuten jeweils zwischen einer Stützfläche und der Zusatzfläche der benachbarten Stützfläche angeordnet sind. Hierdurch wird eine besonders günstige Strömung auf der Auslassseite erzielt.

Bei einem praktischen Ausführungsbeispiel nach der Erfindung ist es bevorzugt, dass die Öffnungen in der Membranscheibe nierenförmig ausgebildet sind.

Hierbei ist es vorteilhaft, dass sechs Stützflächen in dem zweiten Schlauchanschlussgehäuse vorgesehen sind, und dass die Membranscheibe acht Öffnungen aufweist. Hierdurch wird gewährleistet, dass bei der Montage des erfindungsgemäßen Ventils auf jeden Fall eine ausreichende Anzahl von Öffnungen nicht oder nur teilweise durch die Stützflächen bei Überdruck abgedeckt wird.

Im Folgenden wird die Erfindung an Hand einer in den Zeichnungen beispielhaft veranschaulichten Ausführungsform näher erläutert. Es zeigt:

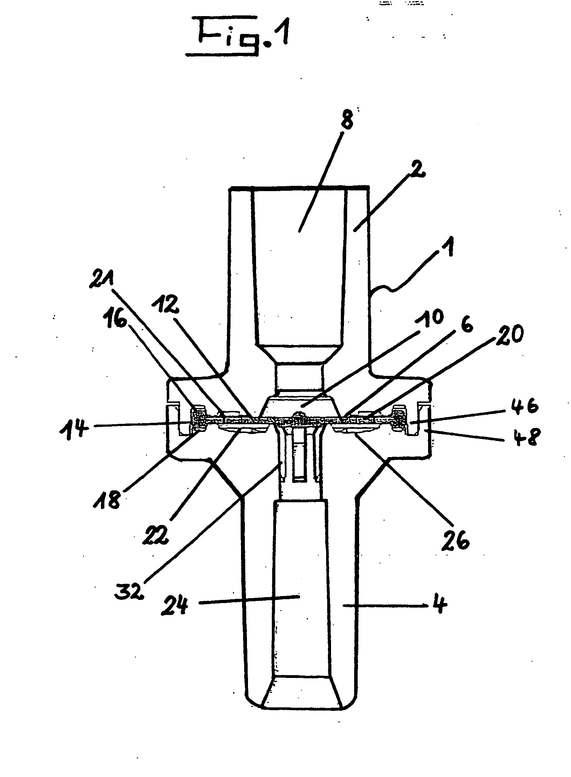

**Figur 1** eine schematische Schnittansicht einer Ausführungsform des Rückschlagventils nach der Erfindung;
**Figur 2** einen stark vergrößerten Ausschnitt des Ventils gemäß Figur 1;
**Figur 3** eine perspektivische Ansicht des zweiten Schlauchanschlussgehäuses des Ventils gemäß Figur 1 und 2 in vergrößerter Darstellung;
**Figur 4** eine teilweise geschnittene Ansicht des Ventils gemäß Figur 3 und
**Figur 5** eine Draufsicht der Darstellung gemäß Figur 3.

Das in den Zeichnungen dargestellte Rückschlagventil 1 ist insbesondere für medizinische Anwendungen geeignet und deckt beispielsweise Druckunterschiede von hohen Drücken bis zu 2,5 bar bis herunter zu 0,5 bar ab. Das Rückschlagventil 1 besteht aus einem ersten Schlauchanschlussgehäuse 2 und einem zweiten Schlauchanschlussgehäuse 4, welche beispielsweise aus Kunststoff durch Spritzgießen hergestellt sind, sowie einer zwischen den beiden Schlauchanschlussgehäusen 2 und 4 angeordneten Membranscheibe 6, welche aus einem flexiblen Kunst-stoff, beispielsweise Silikon, besteht.

Das erste Schlauchanschlussgehäuse 2 weist einen Eingangskanal 8 auf, der in einem Eingangsraum 10 mündet. Der Eingangsraum 10 ist von einem ringförmigen Ventilsitz 12 umgeben, gegen den die Membranscheibe 6 vorgespannt ist.

Die Membranscheibe 6 weist einen durchgehend geschlossenen Mittelteil auf, so dass erhebliche Zugkräfte radial von innen nach außen und umgekehrt übertragen werden können. An ihrem äußeren Umfangsbereich ist die Membranscheibe 6 mit einem Ringwulst 14 versehen, welcher dort beispielsweise an die Membranscheibe 6 angespritzt ist. In dem ersten Schlauchanschlussgehäuse 2 ist eine Ringnut 16 in dessen Stirnfläche ausgebildet, welche eine Ringnut 18 des zweiten Schlauchan-schlussgehäuses 4 im zusammengebauten Zustand gegenüberliegt. Beim Zusammenbau des ersten Schlauchanschlussgehäuses 2 mit dem zweiten Schlauchanschlussgehäuse 4 wird der Ringwulst 14 in den einander gegenüberliegenden Ringnuten 16 und 18 der beiden Schlauchanschlussgehäuse 2 und 4 aufgenommen und gleichzeitig gegen den Ventilsitz 12 vorgespannt.

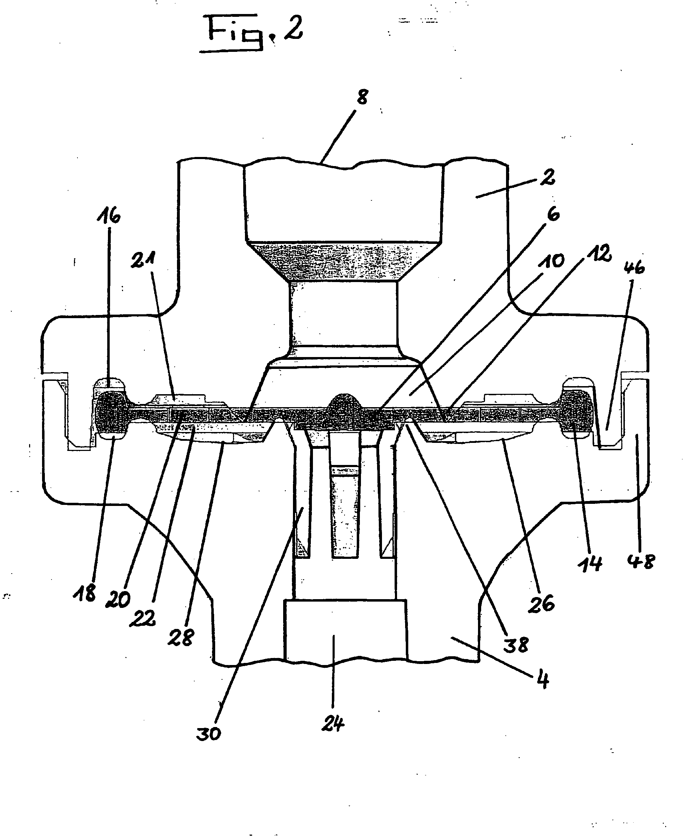

Wie gezeigt, ist die Membranscheibe 6 radial außerhalb des Ventilsitzes 12, wie dies am besten aus Figur 2 ersichtlich ist, mit auf einem Radius angeordneten Öffnungen 20 versehen, welche einen radial außerhalb des Ventilsitzes 12 liegenden Ringraum 21 im ersten Schlauchanschlussgehäuse 2 mit einem Auslassraum 22 im zweiten Schlauchanschlussgehäuse 4 verbinden, der seinerseits mit dem Auslasskanal 24 des zweiten Schlauchanschlussgehäuses 4 verbunden ist.

Der Auslassraum 22 weist eine dem die Öffnungen 20 des Randbereiches gegenüberliegende Wandung 26 auf, in welcher allgemein mit 28 bezeichnete Ausnehmungen vorgesehen sind, die den Öffnungen 20 gegenüberliegen.

Erfindungsgemäß sind die Ausnehmungen 28 durch Stützflächen 30 für die Membranscheibe 6 voneinander getrennt, wobei die Ausnehmungen 28 durch schmale tiefe Nuten 32 mit dem Auslasskanal 24 verbunden sind.

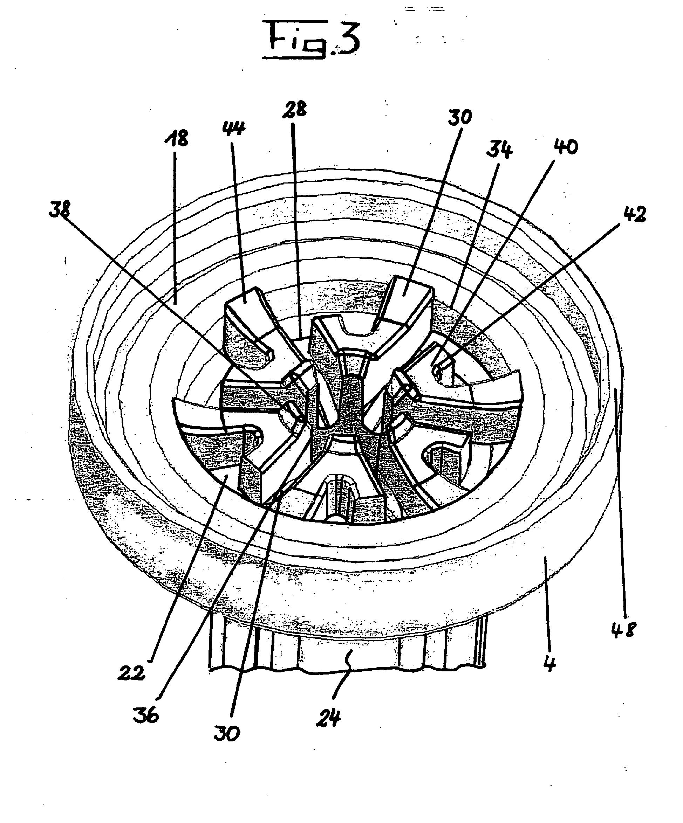

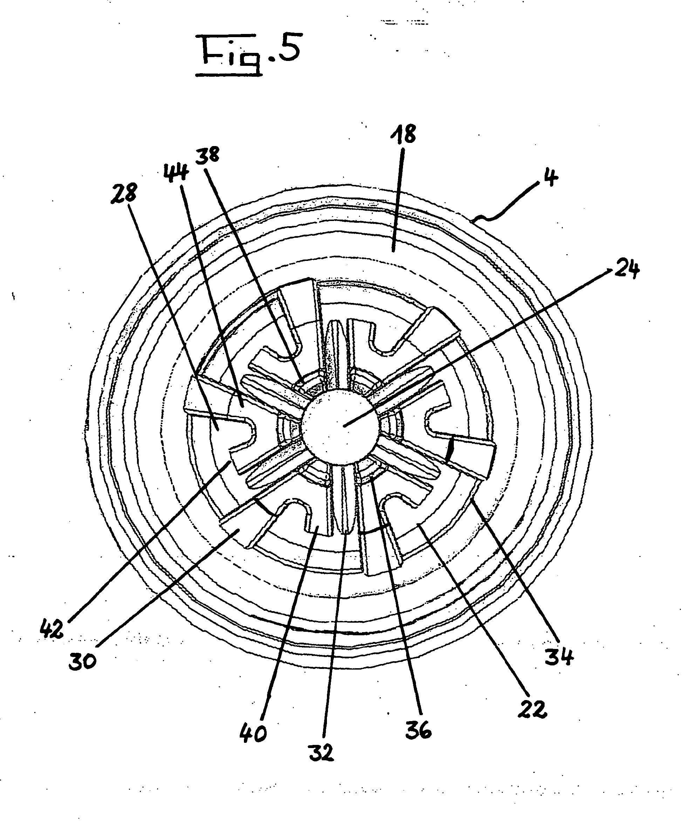

Wie insbesondere aus den Figuren 3 bis 5 ersichtlich, reichen die Stützflächen 30 bis zum Außenrand 34 des Auslassraumes 22. Ferner verbreitern sich die Stützflächen 30 in Richtung des Außenrandes 34 des Auslassraumes, so dass für die mögliche Anlage der Membranscheibe 6 bei hohen Drücken eine größtmögliche Fläche zur Verfügung steht.

Wie ferner insbesondere aus den Figuren 3 bis 5 ersichtlich, weisen die Stützflächen 30 Innenenden 36 auf, welche innerhalb des Radius' des gegenüberliegenden Ventilsitzes angeordnet sind.

Bei einer bevorzugten Ausführungsform, wie sie in den Zeichnungen dargestellt ist, sind die Innenenden 36 der Stützflächen 30 innerhalb des Radius' des Ventilsitzes 12 mit auf die Membranscheibe 6 zuweisenden Vorsprüngen 38 versehen, welche an der Membranscheibe 6 anliegen und diese in Richtung des Einlassraums 10 vorspannen bzw. halten. Die Vorsprünge 38 umgeben daher die vom Auslassraum 22 ausgehende Eingangsöffnung des Auslasskanals 24 ringförmig und in Abständen, wobei die Vorsprünge 38 durch Spritzgießen einstückig mit den Stützflächen 30 ausgebildet sind. Durch die Höhe der Vorsprünge 38 wird auf die Membranscheibe 6 eine gegensinnige Vorspannung ausgeübt, wobei durch Änderung der Höhen der Vorsprünge 38 eine Änderung des Öffnungsdrucks des Rückschlagventils eingestellt werden kann.

Bei der bevorzugten und in den Zeichnungen dargestellten Ausführungsform sind ferner, wie insbesondere aus den Figuren 3 bis 5 ersichtlich, die Stützflächen 30 mit seitlichen, in einem spitzen Winkel abstehenden Zusatzflächen 40 versehen, deren radiale Außenenden 42 innerhalb desjenigen Radius' liegen, auf welchem die Öffnungen 20 in der Membranscheibe 6 angeordnet sind. Hierdurch wird eine zusätzliche Abstützung der Membranscheibe 6 bei hohen Drücken insbesondere im radial innenliegenden Bereich erreicht, welcher besonders beansprucht wird.

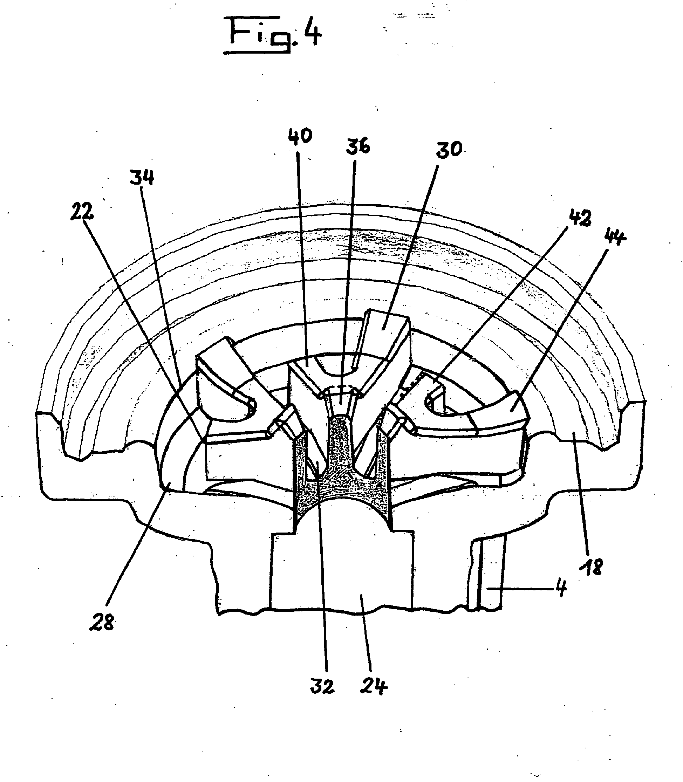

Wie ferner insbesondere aus Figuren 3 und 4 ersichtlich, sind die der Membranscheibe gegenüberliegenden Oberflächen 44 der Stützflächen 30 und der Zusatzflächen 40 konkav gewölbt ausgebildet und entsprechen somit der Form der Membranscheibe 6 bei hohem Druck während des Öffnens des Ventils.

Aus den Figuren 2 bis 5 ist ersichtlich, dass die tiefen und schmalen Nuten 32 jeweils zwischen einer Stützfläche 30 und der Zusatzfläche 40 der benachbarten Stützfläche 30 angeordnet sind, so dass eine möglichst ungestörte Strömung der durch die Öffnungen 20 in den Auslassraum 22 eintretenden Flüssigkeit gewährleistet ist.

Zur Erleichterung der Montage, d.h. um die Notwendigkeit zu vermeiden, die Membranscheibe 6 besonders orientieren zu müssen, sind die Öffnungen 20 in der Membranscheibe 6 nierenförmig ausgebildet. Ferner sind bei der bevorzugten Ausführungsform sechs Stützflächen 30 mit den dazugehörigen Zusatzflächen 40 in dem zweiten Schlauchanschlussgehäuse 4 vorgesehen, wobei die Membranscheibe 6 acht nierenförmige Öffnungen 20 aufweist.

Zur Montage sind die beiden Schlauchanschlussgehäuse 2 und 4 mittels eines innenliegenden Ringvorsprungs 46 am ersten Schlauchanschlussgehäuse mittels eines außenliegenden Ringvorsprungs 48 am zweiten Schlauchanschlussgehäuse 4 ineinandergreifend verbindbar, wobei die endgültige Verbindung dann beispielsweise durch Schweißtechniken, wie Ultraschallschweißen, oder Kleben erfolgen kann.

Sollte nun bei der dargestellten Ausführungsform im Eingangskanal 8 ein starker Überdruck herrschen, durch welchen die Membranscheibe 6 gegen die Wandung 26 gedrückt wird, so legt sie sich gegen die Stützflächen 30 und Zusatzflächen 40 ohne übergroße Dehnung oder Verformung an, so dass die Verbindung zwischen dem Einlassraum 10 und dem Auslassraum 22 erhalten bleibt, da die Öffnungen 20 zum Auslassraum 22 hin offen sind, wobei gleichzeitig jegliche Überdehnung der Membran vermieden wird.

### BEZUGSZEICHENLISTE

- 1 =: Rückschlagventil
- 2 =: erstes Schlauchanschlussgehäuse
- 4 =: zweites Schlauchanschlusssgehäuse
- 6 =: Membranscheibe
- 8 =: Eingangskanal
- 10 =: Einlassraum
- 12 =: Ventilsitz
- 14 =: Ringwulst
- 16 =: Ringnut
- 18 =: Ringnut
- 20 =: Öffnung
- 21 =: Ringraum
- 22 =: Auslassraum
- 24 =: Auslasskanal
- 26 =: Wandung
- 28 =: Ausnehmung
- 30 =: Stützfläche
- 32 =: Nut
- 34 =: Außenrand v. 22
- 36 =: Innenende v. 30
- 38 =: Vorsprung
- 40 =: Zusatzfläche
- 42 =: Außenende v. 40
- 44 =: Oberfläche v. 30 bzw. 40
- 46 =: Ringvorsprung innen
- 48 =: Ringvorsprung außen

## Patentansprüche

1. Rückschlagventil, insbesondere für medizinische Anwendungen, mit einem ersten Schlauchanschlussgehäuse (2) und einem zweiten Schlauchanschlussgehäuse (4) und einer zwischen den beiden Schlauchanschlussgehäusen angeordneten Membranscheibe (6) aus flexiblem Werkstoff, welche bei Überdruck in einem Eingangskanal (8) des ersten Schlauchanschlussgehäuses (2) von einem ringförmigen, einen mit dem Eingangskanal verbundenen Einlassraum (10) umgebenden Ventilsitz (12) abhebbar ist und die bei Überdruck in einem Auslasskanal (24) des zweiten Schlauchanschlussgehäuses (4) sicher und in Minimalzeiten auf den Ventilsitz (12) andrückbar ist, wobei die Membranscheibe (6) in ihrem äußeren Umfangsbereich mit einem Ringwulst (14) versehen ist, der in einander gegenüberliegenden Ringnuten (16, 18) der Schlauchanschlussgehäuse aufgenommen ist, die Membranscheibe (6) radial außerhalb des Ventilsitzes mit zu einem Auslassraum (22) führenden Öffnungen (20) versehen ist und wobei die den Öffnungen gegenüberliegende Wandung (26) des zweiten Schlauchanschlussgehäuses (4) mit mit dem Auslasskanal (24) in Verbindung stehenden Ausnehmungen (28) versehen ist, **dadurch gekennzeichnet, dass** die Ausnehmungen (28) durch Stützflächen (30) für die Membranscheibe (6) voneinander getrennt sind, und dass die Ausnehmungen (28) durch schmale tiefe Nuten (32) mit dem Auslasskanal (24) verbunden sind.

2. Rückschlagventil nach Anspruch 1, **dadurch gekennzeichnet, dass** die Stützflächen (30) bis zum Außenrand (34) des Auslassraums (22) reichend ausgebildet sind.

3. Rückschlagventil nach Anspruch 1 oder 2, **dadurch gekennzeichnet, dass** die Stützflächen (30) sich in Richtung des Außenrandes (34) des Auslassraums (22) verbreiternd ausgebildet sind.

4. Rückschlagventil nach einem der vorstehenden Ansprüche, **dadurch gekennzeichnet, dass** die Stützflächen (30) Innenenden (36) aufweisen, welche innerhalb des Radius des gegenüberliegenden Ventilsitzes (12) angeordnet sind.

5. Rückschlagventil nach Anspruch 4, **dadurch gekennzeichnet, dass** die Innenenden (36) mit etwa auf deren Radius angeordneten Vorsprüngen (38) versehen sind, die an der Membranscheibe (6) anliegen und diese in Richtung des Einlassraums (10) halten bzw. vorspannen.

6. Rückschlagventil nach einem der vorstehenden Ansprüche, **dadurch gekennzeichnet, dass** die Stützflächen (30) mit seitlich in einem spitzen Winkel abstehenden Zusatzflächen (40) versehen sind, deren radiale Außenenden (42) innerhalb des Radius liegen, auf welchem die Öffnungen (20) in der Membranscheibe (6) angeordnet sind.

7. Rückschlagventil nach einem der vorstehenden Ansprüche, **dadurch gekennzeichnet, dass** die der Membranscheibe (6) gegenüberliegende Oberfläche (44) der Stützflächen (30) und/oder Zusatzflächen (40) konkav gewölbt ausgebildet ist.

8. Rückschlagventil nach einem der vorstehenden Ansprüche, **dadurch gekennzeichnet, dass** die Nuten (32) jeweils zwischen einer Stützfläche (30) und der Zusatzfläche (40) der benachbarten Stützfläche (30) angeordnet sind.

9. Rückschlagventil nach einem der vorstehenden Ansprüche, **dadurch gekennzeichnet, dass** die Öffnungen (20) in der Membranscheibe (6) nierenförmig ausgebildet sind.

10. Rückschlagventil nach einem der vorstehenden Ansprüche, **dadurch gekennzeichnet, dass** sechs Stützflächen (30) in dem zweiten Schlauchanschlussgehäuse (4) vorgesehen sind, und dass die Membranscheibe (6) acht Öffnungen (20) aufweist.

## Claims

1. Nonreturn valve, in particular for medical applications, with a first tube connector housing (2) and a second tube connector housing (4), and with a membrane disc (6) which is made of flexible material and is arranged between the two tube connector housings and which, when there is an overpressure in an inlet channel (8) of the first tube connector housing (2), can be lifted from an annular valve seat (12) surrounding an inlet chamber (10) connected to the inlet channel, and which, when there is an overpressure in an outlet channel (24) of the second tube connector housing (4), can be pressed firmly and within minimal times onto the valve seat (12), the membrane disc (6) is provided in its outer peripheral area with an annular bead (14) which is received in mutually opposite annular grooves (16, 18) of the tube connector housings, the membrane disc (6) is provided, radially outside the valve seat, with openings (20) that lead to an outlet chamber (22), and the wall (26) of the second tube connector housing (4) lying opposite the openings (22) is provided with recesses (28) communicating with the outlet channel (24), **characterized in that** the recesses (28) are separated from one another by support surfaces (30) for the membrane disc (6), and **in that** the recesses (28) are connected to the outlet channel (24) via narrow deep grooves (32).

2. Nonreturn valve according to Claim 1, **characterized in that** the support surfaces (30) reach as far as the outer edge (34) of the outlet chamber (22).

3. Nonreturn valve according to Claim 1 or 2, **characterized in that** the support surfaces (30) are designed widening in the direction of the outer edge (34) of the outlet chamber (22).

4. Nonreturn valve according to one of the preceding claims, **characterized in that** the support surfaces (30) have inner ends (36) arranged within the radius of the opposite valve seat (12).

5. Nonreturn valve according to Claim 4, **characterized in that** the inner ends (36) are provided with projections (38), which are arranged approximately on the radius thereof and which bear on the membrane disc (6) and hold or pretension the latter in the direction of the inlet chamber (10).

6. Nonreturn valve according to one of the preceding claims, **characterized in that** the support surfaces (30) are provided with auxiliary surfaces (40), which protrude laterally at an acute angle and whose radial outer ends (42) lie within the radius on which the openings (20) in the membrane disc (6) are arranged.

7. Nonreturn valve according to one of the preceding claims, **characterized in that** the face (44) of the support surfaces (30) lying opposite the membrane disc (6) and/or the auxiliary surfaces (40) are designed with a concave curvature.

8. Nonreturn valve according to one of the preceding claims, **characterized in that** the grooves (32) are each arranged between a support surface (30) and the auxiliary surface (40) of the adjacent support surface (30).

9. Nonreturn valve according to one of the preceding claims, **characterized in that** the openings (20) in the membrane disc (6) are kidney-shaped.

10. Nonreturn valve according to one of the preceding claims, **characterized in that** six support surfaces (30) are provided in the second tube connector housing (4), and **in that** the membrane disc (6) has eight openings (20).

## Revendications

1. Clapet anti-retour, en particulier pour des applications médicales, comprenant un premier boîtier de raccordement de tuyau (2) et un deuxième boîtier de raccordement de tuyau (4) ainsi qu'un disque à membrane (6) en matériau flexible disposé entre les deux boîtiers de raccordement de tuyau, qui, en cas de surpression dans un canal d'entrée (8) du premier boîtier de raccordement de tuyau (2), peut se soulever d'un siège de clapet annulaire (12), entourant un espace d'entrée (10) connecté au canal d'entrée et qui, en cas de surpression dans un canal de sortie (24) du deuxième boîtier de raccordement de tuyau (4), peut être pressé de manière sûre et dans un temps minimum, sur le siège de clapet (12), le disque à membrane (6) étant pourvu, dans sa région périphérique extérieure, d'un bourrelet annulaire (14) qui est reçu dans des rainures annulaires opposées (16, 18) des boîtiers de raccordement de tuyau, le disque à membrane (6) étant pourvu, radialement en dehors du siège de clapet, d'ouvertures (20) conduisant à un espace de sortie (22) et la paroi (26) du deuxième boîtier de raccordement de tuyau (4) opposée aux ouvertures étant pourvue d'évidements (28) en liaison avec le canal de sortie (24), **caractérisé en ce que** les évidements (28) sont séparés les uns des autres par des surfaces de support (30) pour le disque à membrane (6), et **en ce que** les évidements (28) sont connectés par des rainures étroites et profondes (32) au canal de sortie (24).

2. Clapet anti-retour selon la revendication 1, **caractérisé en ce que** les surfaces de support (30) sont réalisées de manière à s'étendre jusqu'au bord extérieur (34) de l'espace de sortie (22).

3. Clapet anti-retour selon la revendication 1 ou 2, **caractérisé en ce que** les surfaces de support (30) sont réalisées de manière à s'élargir dans la région du bord extérieur (34) de l'espace de sortie (22).

4. Clapet anti-retour selon l'une quelconque des revendications précédentes, **caractérisé en ce que** les surfaces de support (30) présentent des extrémités internes (36) qui sont disposées à l'intérieur du rayon du siège de clapet (12) opposé.

5. Clapet anti-retour selon la revendication 4, **caractérisé en ce que** les extrémités internes (36) sont pourvues de saillies (38) disposées approximativement sur leur rayon, qui s'appliquent contre le disque à membrane (6) et qui le maintiennent ou le précontraignent dans la direction de l'espace d'entrée (10).

6. Clapet anti-retour selon l'une quelconque des revendications précédentes, **caractérisé en ce que** les surfaces de support (30) sont pourvues de surfaces supplémentaires (40) saillant latéralement suivant un angle aigu, dont les extrémités extérieures radiales (42) se situent à l'intérieur du rayon sur lequel sont disposées les ouvertures (20) dans le disque à membrane (6).

7. Clapet anti-retour selon l'une quelconque des revendications précédentes, **caractérisé en ce que** la surface (44) des surfaces de support (30) et/ou des surfaces supplémentaires (40) opposée au disque à membrane (6) est réalisée avec une courbure concave.

8. Clapet anti-retour selon l'une quelconque des revendications précédentes, **caractérisé en ce que** les rainures (32) sont à chaque fois disposées entre une surface de support (30) et la surface supplémentaire (40) de la surface de support (30) adjacente.

9. Clapet anti-retour selon l'une quelconque des revendications précédentes, **caractérisé en ce que** les ouvertures (20) sont disposées en forme de haricot dans le disque à membrane (6).

10. Clapet anti-retour selon l'une quelconque des revendications précédentes, **caractérisé en ce que** six surfaces de support (30) sont prévues dans le deuxième boîtier de raccordement de tuyau (4) et **en ce que** le disque à membrane (6) présente huit ouvertures (20).
